# EUROPEAN PATENT SPECIFICATION

(11) **EP 1 434 582 B1**
(45) Date of publication and mention of the grant of the patent: **13.04.2005**
(21) Application number: 02779378.5
(22) Date of filing: 18.09.2002
(51) Int. Cl.: A61K 31/475, A61K 47/12, A61P 17/02

(54) **TOPICAL COMPOSITION CONTAINING BRUCINE AND THE USE FOR THE TREATMENT OF DAMAGED MAMMALIAN SKIN**
TOPISCHE ZUSAMMENSETZUNG ENTHALTEND BRUCIN UND IHRE VERWENDUNG ZUR BEHANDLUNG VON HAUTSCHÄDEN
COMPOSITION TOPIQUE COMPRENANT BRUCINE

(30) Priority: 20.09.2001 IE 20010841
(43) Date of publication of application: 07.07.2004
(73) Proprietor: Boyle, Frank, Ballinfull, Co. Sligo (IE)
(72) Inventor: VILA, Anthony, Mazatlan, Sinaloa 82100 (MX)
(74) Representative: Duffy, Assumpta Dympna
(86) International application number: PCT/EP2002/010522
(87) International publication number: WO 2003/024454

(56) References cited:
- US-A- 3 531 570
- US-A- 5 747 021
- DATABASE WPI Section Ch, Week 200268 Derwent Publications Ltd., London, GB; Class B05, AN 2002-628780 XP002234267 & CN 1 355 021 A (ZHANG X), 26 June 2002 (2002-06-26)
- DATABASE WPI Section Ch, Week 199708 Derwent Publications Ltd., London, GB; Class A96, AN 1997-083374 XP002234268 & JP 08 325131 A (KYOWA HAKKO KOGYO KK), 10 December 1996 (1996-12-10)

## Description

This invention relates to a topical composition. In particular, it relates to a dermatological composition suitable for topical application to mammalian skin. Such composition is useful in the cosmetic or medical treatment of damaged mammalian skin, especially damage caused by sunburn, minor burns, ulcers or the like.

Brucine (2,3-dimethoxystrychnidin-10-one)is a highly toxic alkaloid resembling strychnine, which can be used as an addition agent for lubricants.

It has now unexpectedly been found that brucine or a dermatologically acceptable derivative thereof is useful in the regeneration of mammalian tissue.

According to the present invention there is provided the use of brucine or a dermatologically acceptable salt thereof, or a hydrate thereof, in the manufacture of a medicament for the treatment of damaged mammalian skin.

The invention also provides a dermatological composition suitable for topical application to mammalian skin, which comprises:
(a) 0.05-0.3% by weight of brucine or a dermatologically acceptable salt thereof, or a hydrate thereof;
(b) 0.8-3% by weight of a hydroxy acid or a salt, ester or amide thereof;
(c) 0.15-5% by weight of water; and
(d) the balance comprising a dermatologically acceptable oil or water or a combination thereof.

In the present invention, brucine is preferably used in the form of a salt, more preferably its sulphate salt. In a particularly preferred embodiment, brucine is used in the form of brucine sulphate heptahydrate. Brucine or a salt thereof or a hydrate thereof is preferably present in the composition in an amount of from 0.1-0.2%, especially 0.15%, by weight of the composition.

The hydroxy acid used in the composition of the present invention may be any suitable hydroxy acid, such as, for example, glycolic, lactic, malic, tartaric, citric, mandelic or salicylic acid or a mixture thereof. The hydroxy acid may be present in the form of the free acid and/or in the form of one of its associated salts, especially salts with an organic base or an alkali metal, or in the form of an ester or amide thereof. Sodium salicylate is particularly preferred. The hydroxy acid is preferably present in the composition in an amount of from 1.0-2.5%, more preferably 1.2-1.8%, and especially 1.5%, by weight of the composition.

Component (c) of the composition comprises water in an amount of from 0.15-5%, preferably from 0.5-3%, more preferably from 0.75-2.5%, and especially 2%, by weight of the composition.

Component (d) of the composition comprises a dermatologically acceptable oil, or water, or a combination thereof. In a preferred embodiment, the composition is in the form of a water-in-oil emulsion. In a particularly preferred embodiment, component (d) consists of a dermatologically acceptable oil. Any suitable oil may be used, such as mineral oil or a vegetable oil, e.g. sunflower oil, or a mixture thereof. Pure mineral oil is preferred. In a particularly preferred embodiment, the mineral oil is free from preservative.

In a further preferred embodiment, component (d) of the composition consists of water.

The composition of the invention may also include a masking agent such as caffeine or the like, preferably in an amount of from 0.03-0.08%, especially 0.05%, by weight of the composition.

The composition of the invention may be formed by (a) heating the brucine or salt thereof or hydrate thereof in a suitable solvent, such as an alcohol, preferably ethanol or isopropanol, until the brucine is dissolved; (b) adding the hydroxy acid and masking agent (if used) to the water or a portion thereof and heating to a temperature in the range of from 70°C to 85°C until they are dissolved; (c) heating the oil, if present, to a temperature in the range of from 70°C to 85°C; (d) mixing the brucine and hydroxy acid solutions and any of the water remaining, or, if oil is present, adding the brucine and hydroxy acid solutions and any of the water remaining, to the heated oil; and (e) heating the resulting product to a temperature in the range of from 80°C to 95°C so as to evaporate substantially all of the solvent. The resulting product may then be homogenised until fully blended.

The invention will be more clearly understood from the following description thereof given by way of example only.

### EXAMPLE 1

| **Ingredients** | **% by weight** |
|---|---|
| Brucine sulphate heptahydrate | 0.15 |
| Sodium salicylate | 1.5 |
| Distilled water | 2.0 |
| Caffeine | 0.05 |
| Mineral oil (preservative-free) | qv100 |

A water-in-oil emulsion was prepared by adding the sodium salicylate and caffeine to the water and heating to approximately 80°C for 1-2 minutes until clear. The brucine sulphate heptahydrate was dissolved in approximately 20.0 ml of ethanol by heating to a temperature of approximately 80°C. The mineral oil was heated to approximately 80°C and the sodium salicylate/caffeine and brucine solutions were added thereto. The resulting product was heated to approximately 90°C for approximately 5 - 10 minutes to evaporate the ethanol. The product was then homogenised until fully blended.

### EXAMPLE 2

| **Ingredients** | **% by weight** |
|---|---|
| Brucine sulphate heptahydrate | 0.15 |
| Sodium salicylate | 1.5 |
| Caffeine | 0.05 |
| Distilled Water | qv100 |

An aqueous formulation was prepared by adding the sodium salicylate and caffeine to the water and heating to approximately 80°C for 1-2 minutes until clear. The brucine sulphate heptahydrate was dissolved in approximately 20.0 ml of ethanol by heating to a temperature of approximately 80°C. The sodium salicylate/caffeine and brucine solutions were mixed together and heated to approximately 90°C for approximately 5 - 10 minutes to evaporate the ethanol.

The compositions prepared in Examples 1 and 2 were found to be effective in reducing trauma and regenerating damaged skin resulting from sunburn, minor burns and ulcers.

The results of a clinical study carried out using the emulsion of Example 1 are shown in Table I.

**TABLE I**

| **PATIENT NUMBER/GENDER** | **AGE** | **OCCUPATION** | **TYPE OF BURN** | **SEVERITY OF BURN (1-5)*** | **TIME FROM INJURY TO TREATMENT** | **APPLICATION** | **RESULTS AND OBSERVATIONS** |
|---|---|---|---|---|---|---|---|
| 1. Female | 55 | Secretary | Hot Water | 3 | 1 hour | Every 5 to 10 minutes for 3 hours | Pain gone in 10 minutes. Redness gone in 20 minutes. Completely healed by 3^{rd} hour with no scarring. No further application needed |
| 2. Female | 41 | Receptionist | Curling Iron | 2 | 30 Minutes | Every 5 to 10 minutes for 4 hours | Pain gone by second application. Redness gone by 1^{st} hour. No scarring. |
| 3. Female | 39 | Lab Tech | Acid Burn | 5 | 5 Minutes | Every 10 minutes for 1 day | Pain gone by 2^{nd} hour. Most redness gone by 3^{rd} hour. Product applied throughout 1^{st} day. Redness greatly reduced on day 2. No pain on 2^{nd} day. No scarring noticed |
| 4. Male | 25 | Construction Worker | Sunburn | 2 | 2 hours | Every 10 minutes for 1 day. Two applications next morning. | Pain gone by 1^{st} Hour. Redness redacted to 75% by next morning. No scarring. No visible injury on 3^{rd} day. |
| 5. Male | 41 | Firefighter | Fire | 3 | 4 hours | Every 10 minutes for 1 day. Once an hour the next day. | Pain gone by 4^{th} application. Redness reduced by 50% next morning. Redness completely gone by end of second day. No pain on second day. No scarring noticed after one week. |
| 6. Female | 43 | Billing Agent | Hot engine | 3 | 2 hours | Every 10 to 15 minutes for 1 day | Most pain gone by 1^{st} hour. All pain gone by end of day. Redness gone by 4^{th} hour. No sign of scarring noticed after 2^{nd} day |
| 7. Male | 14 | Student | Friction burn from rope | 2 | 3 hours | Every 10 minutes for 1½ days. | Pain gone by 1^{st} hour. Redness cut in half by 4^{th} application. No scarring noticed one week later. |
| 8. Female | 27 | Office Worker | Severe sunburn | 5 | 3½ days | Every 15 to 30 minutes for 2 days | Pain reduced by 50% after 4^{th} hour. Pain completely gone by end 1^{st} day. Normal activities resumed on second day. Redness reduced by 50-% by end of 1^{st} day. Age spots appeared due to severity of burn. Able to return work on next day. completely healed by 4^{th} day. |

- Severity of burn indicates a standard used by those involved in this clinical study to identify the severity of a burn, 1 being least painful and 5 being most painful.
- The amount of formulation applied was sufficient to cover the area being treated.
- Patient No. 8 had previously been to three other doctors and had not found success with any treatment until she had used the product of Example 1. She had not been to work for three days.
- Patients in this clinical study were all able to return to work or school with no problems after using the product of Example 1.

## Claims

1. The use of brucine (2,3-dimethoxystrychnidin-10-one)or a dermatologically acceptable salt thereof, or a hydrate thereof, in the manufacture of a medicament for the treatment of damaged mammalian skin.

2. Use according to claim 1 wherein brucine is in the form of brucine sulphate heptahydrate.

3. A dermatological composition suitable for topical application to mammalian skin, which comprises:
(a) 0.05-0.3% by weight of brucine or a dermatologically acceptable salt thereof, or a hydrate thereof;
(b) 0.8-3% by weight of a hydroxy acid or a salt, ester or amide thereof;
(c) 0.15-5% by weight of water; and
(d) the balance comprising a dermatologically acceptable oil or water or a combination thereof.

4. A composition according to claim 3, wherein brucine is in the form of brucine sulphate heptahydrate.

5. A composition according to claim 3 or 4, wherein brucine or a salt thereof or a hydrate thereof is present in an amount of from 0.1-0.2%, especially 0.15% by weight of the composition.

6. A composition according to one or more of claims 3 to 5, wherein the hydroxy acid is selected from glycolic, lactic, malic, tartaric, citric, mandelic or salicylic acid, or a salt thereof, or a mixture thereof, preferably sodium salicylate.

7. A composition according to one or more of claims 3 to 6, wherein the hydroxy acid or salt, ester or amide thereof is present in an amount of from 1.0-2.5%, preferably 1.2-1.8%, and especially 1.5%, by weight of the composition.

8. A composition according to one or more of claims 3 to 7, wherein component (c) of the composition comprises water in an amount of from 0.5-3%, preferably from 0.75-2.5%, and especially 2%, by weight of the composition.

9. A composition according to one or more of claims 3 to 8, wherein component (d) consists of water.

10. A composition according to one or more of claims 3 to 8, which is in the form of a water-in-oil emulsion, wherein preferably, component (d) consists of a dermatologically acceptable oil.

11. A composition according to one or more of claims 3 to 8 or 10, wherein the dermatologically acceptable oil is mineral oil or a vegetable oil, or a mixture thereof.

12. A composition according to claim 11, wherein the oil is mineral oil, preferably mineral oil which is free from preservative.

13. A composition according to one or more of the preceding claims, further comprising a masking agent, preferably caffeine, the masking agent preferably being present in an amount of from 0.03-0.08%, especially 0.05%, by weight of the composition.

14. A composition according to one or more of claims 3 to 13 for use in the treatment of damaged mammalian skin.

15. A method of preparing a composition according to one or more of claims 3 to 13, comprising the following steps:
(a) heating the brucine or salt thereof or hydrate thereof in a solvent, preferably ethanol, until the brucine is dissolved;
(b) adding the hydroxy acid and masking agent, if present, to the water or a portion thereof and heating to a temperature in the range of from 70°C to 85°C until they are dissolved;
(c) heating the oil, if present, to a temperature in the range of from 70°C to 85°C;
(d) mixing the brucine and hydroxy acid solutions and any of the water remaining, or, if oil is present, adding the brucine and hydroxy acid solutions and any of the water remaining, to the heated oil; and
(e) heating the resulting product to a temperature in the range of from 80°C to 95°C so as to evaporate substantially all of the solvent.

## Patentansprüche

1. Verwendung von Brucin (2,3-Dimethoxystrychnidin-10-on) oder einem dermatologisch verträglichen Salz davon oder einem Hydrat davon, bei der Herstellung eines Arzneimittels zur Behandlung von geschädigter Haut bei Säugern.

2. Verwendung nach Anspruch 1, worin Brucin in der Form von Brucinsulfat-Heptahydrat vorliegt.

3. Dermatologische Zusammensetzung, die zur topischen Applikation auf die Haut von Säugern geeignet ist, die Folgendes umfasst:
(a) 0,05 - 0,3 Gew.-% Brucin oder ein dermatologisch verträgliches Salz davon oder ein Hydrat davon;
(b) 0,8 - 3 Gew.-% einer Hydroxysäure oder eines Salzes, Esters oder Amids davon;
(c) 0,15 - 5 Gew.-% Wasser; und
(d) der Rest umfassend ein dermatologisch verträgliches Öl oder Wasser oder eine Kombination davon.

4. Zusammensetzung nach Anspruch 3, worin Brucin in der Form von Brucinsulfat-Heptahydrat vorliegt.

5. Zusammensetzung nach Anspruch 3 oder 4, worin Brucin oder ein Salz davon oder ein Hydrat davon in einer Menge von 0,1 - 0,2 Gew.-%, insbesondere 0,15 Gew.-% der Zusammensetzung vorliegt.

6. Zusammensetzung nach einem oder mehreren der Ansprüche 3 bis 5, worin die Hydroxysäure aus Glycol-, Milch-, Äpfel-, Wein-, Citronen-, Mandel- oder Salicylsäure oder einem Salz davon, oder einem Gemisch davon, bevorzugt Natriumsalicylat, ausgewählt ist.

7. Zusammensetzung nach einem oder mehreren der Ansprüche 3 bis 6, worin die Hydroxysäure oder das Salz, der Ester oder das Amid davon in einer Menge von 1,0 - 2,5 Gew.-%, bevorzugt 1,2 - 1,8 Gew.-% und insbesondere 1,5 Gew.-% der Zusammensetzung vorliegt.

8. Zusammensetzung nach einem oder mehreren der Ansprüche 3 bis 7, worin Komponente (c) der Zusammensetzung Wasser in einer Menge von 0,5 - 3 Gew.-%, bevorzugt von 0,75 - 2,5 Gew.-% und insbesondere 2 Gew.-% der Zusammensetzung umfasst.

9. Zusammensetzung nach einem oder mehreren der Ansprüche 3 bis 8, worin Komponente (d) aus Wasser besteht.

10. Zusammensetzung nach einem oder mehreren der Ansprüche 3 bis 8, die in der Form einer Wasser-in-Öl-Emulsion vorliegt, worin Komponente (d) bevorzugt aus einem dermatologisch verträglichen Öl besteht.

11. Zusammensetzung nach einem oder mehreren der Ansprüche 3 bis 8 oder 10, worin das dermatologisch verträgliche Öl Mineralöl oder ein pflanzliches Öl oder ein Gemisch davon darstellt.

12. Zusammensetzung nach Anspruch 11, worin das Öl Mineralöl, bevorzugt Mineralöl, das konservierungsmittelfrei ist, darstellt.

13. Zusammensetzung nach einem oder mehreren der vorangehenden Ansprüche, weiter umfassend ein Maskierungsmittel, bevorzugt Coffein, wobei das Maskierungsmittel bevorzugt in einer Menge von 0,03 - 0,08 Gew.-%, insbesondere 0,05 Gew.-% der Zusammensetzung, vorliegt.

14. Zusammensetzung nach einem oder mehreren der Ansprüche 3 bis 13 zur Verwendung bei der Behandlung von geschädigter Haut bei Säugern.

15. Verfahren zur Herstellung einer Zusammensetzung nach einem oder mehreren der Ansprüche 3 bis 13, umfassend die folgenden Schritte:
(a) Erhitzen des Brucins oder Salzes davon oder Hydrats davon, in einem Lösungsmittel, bevorzugt Ethanol, bis das Brucin aufgelöst ist;
(b) Zufügen der Hydroxysäure und gegebenenfalls des Maskierungsmittels zum Wasser oder einem Anteil davon, und Erhitzen auf eine Temperatur im Bereich von 70 °C bis 85 °C, bis sie aufgelöst sind;
(c) gegebenenfalls Erhitzen des Öls auf eine Temperatur im Bereich von 70 °C bis 85 °C;
(d) Mischen des Brucins und der Hydroxysäure-Lösungen und jedwedes des übrigen Wassers oder, wenn Öl vorliegt, Zufügen des Brucins und der Hydroxysäure-Lösungen und jedwedes des übrigen Wassers, zum erhitzten Öl; und
(e) Erhitzen des sich ergebenden Produkts auf eine Temperatur im Bereich von 80 °C bis 95 °C, um auf diese Weise weitgehend das gesamte Lösungsmittel zu verdampfen.

## Revendications

1. Utilisation de brucine (2,3-diméthoxystrychnidin-10-one) ou d'un sel dermatologiquement acceptable de celle-ci, ou d'un hydrate de celui-ci, dans la fabrication d'un médicament pour le traitement des dommages de la peau de mammifères.

2. Utilisation selon la revendication 1, dans laquelle la brucine est sous forme de sulfate de brucine heptahydraté.

3. Composition dermatologique appropriée pour une application topique sur la peau de mammifères comprenant :
(a) de 0,05 % à 0,3% en poids de brucine ou d'un sel dermatologiquement acceptable de celle-ci, ou d'un hydrate de celui-ci ;
(b) de 0,8% à 3% en poids d'un hydroxyacide ou d'un sel, ester ou amide de celui-ci ;
(c) de 0,15 % à 5% en poids d'eau ; et
(d) le reste comprenant une huile dermatologiquement acceptable ou de l'eau, ou une combinaison de celles-ci.

4. Composition selon la revendication 3, dans laquelle la brucine est sous la forme de sulfate de brucine heptahydraté.

5. Composition selon les revendications 3 ou 4, dans laquelle la brucine ou un sel de celle-ci ou un hydrate de celui-ci est présent dans une quantité de 0,1% à 0,2% en poids, particulièrement de 0,15% en poids de la composition.

6. Composition selon l'une ou plusieurs des revendications 3 à 5, dans laquelle l'hydroxyacide est choisi parmi l'acide glycolique, l'acide lactique, l'acide malique, l'acide tartrique, l'acide citrique, l'acide mandélique ou l'acide salicylique, ou leurs sels, ou leurs mélanges, de préférence le salicylate de sodium.

7. Composition selon l'une ou plusieurs des revendications 3 à 6, dans laquelle l'hydroxyacide ou son sel, ester ou amide est présent dans une quantité de 1,0% à 2,5%, de préférence de 1,2% à 1,8%, et particulièrement de 1,5% en poids de la composition.

8. Composition selon l'une ou plusieurs des revendications 3 à 7, dans laquelle le composant (c) de la composition comprend de l'eau dans une quantité de 0,5% à 3%, de préférence de 0,75% à 2,5%, et particulièrement de 2% en poids de la composition.

9. Composition selon l'une ou plusieurs des revendications 3 à 8, dans laquelle le composant (d) consiste en eau.

10. Composition selon l'une ou plusieurs des revendications 3 à 8, qui est sous la forme d'une émulsion eau-dans-huile, dans laquelle le composant (d) consiste de préférence en une huile dermatologiquement acceptable.

11. Composition selon l'une ou plusieurs des revendications 3 à 8 ou 10, dans laquelle l'huile dermatologiquement acceptable est une huile minérale ou une huile végétale, ou leur mélange.

12. Composition selon la revendication 11, dans laquelle l'huile est une huile minérale, de préférence une huile minérale sans conservateur.

13. Composition selon l'une ou plusieurs des revendications précédentes, comprenant en outre un agent de masquage, de préférence la caféine, l'agent de masquage étant de préférence présent dans une quantité de 0,03% à 0,08%, particulièrement de 0,05% en poids de la composition.

14. Composition selon l'une ou plusieurs des revendications 3 à 13 pour une utilisation dans le traitement des dommages de la peau de mammifères.

15. Procédé de préparation d'une composition selon l'une ou plusieurs des revendications 3 à 13, comprenant les étapes suivantes consistant à :
(a) chauffer la brucine ou son sel ou son hydrate dans un solvant, de préférence l'éthanol, jusqu'à dissolution de la brucine ;
(b) ajouter l'hydroxyacide et l'agent masquant, le cas échéant, à l'eau ou à une partie de celle-ci et à chauffer à une température de 70°C à 85°C jusqu'à leur dissolution ;
(c) chauffer l'huile, le cas échéant, à une température de 70°C à 85°C ;
(d) mélanger les solutions de brucine et d'hydroxyacide et l'eau restante ou, si de l'huile est utilisée, ajouter les solutions de brucine et d'hydroxyacide et l'eau restante à l'huile chauffée ; et
(e) chauffer le produit résultant à une température de 80°C à 95°C de manière à évaporer principalement tout le solvant.
